# EUROPÄISCHE PATENTSCHRIFT

(11) **EP 0 888 276 B1**
(45) Veröffentlichungstag und Bekanntmachung des Hinweises auf die Patenterteilung: **06.02.2002**
(21) Anmeldenummer: 97908182.5
(22) Anmeldetag: 06.03.1997
(51) Int. Cl.: C07C 49/683, C07C 49/753, C07C 49/697, C07C 225/22, A61K 7/42, A61K 31/125, C07C 45/51

(54) **CINNAMYLIDENCAMPHERDERIVATE UND IHRE VERWENDUNG ALS UV-A LICHTSCHUTZMITTEL**
CINNAMYLIDENE CAMPHOR DERIVATIVES AND THEIR USE AS UV-A PROTECTING AGENTS
DERIVES DU CINNAMYLIDENE-CAMPHRE ET LEUR UTILISATION COMME AGENTS DE PROTECTION CONTRE LES UV-A

(30) Priorität: 13.03.1996 DE 19609900; 04.09.1996 DE 19635781
(43) Veröffentlichungstag der Anmeldung: 07.01.1999
(73) Patentinhaber: BASF AKTIENGESELLSCHAFT, 67056 Ludwigshafen (DE)
(72) Erfinder: KANDZIA, Christof, D-67157 Wachenheim (DE); WESTENFELDER, Horst, D-67435 Neustadt (DE); SCHEHLMANN, Volker, D-67354 Römerberg (DE)
(86) Internationale Anmeldenummer: EP9701122
(87) Internationale Veröffentlichungsnummer: WO9733855

(56) Entgegenhaltungen:
- DE-A- 2 051 824
- DE-A- 2 336 219
- DE-A- 3 445 712
- CHEMICAL ABSTRACTS, vol. 97, no. 11, 13.September 1982 Columbus, Ohio, US; abstract no. 91857b, S.M. MAKIN ET AL.: "Chemistry of enol ethers. LVI. Study of the condensation of acetals of aromatic aldehydes with 1-ethoxy-1,3-butadiene. Synthesis of arylpolyene aldehydes" Seite 732; XP002036894 & ZH. ORG. KHIM., Bd. 18, Nr. 4, 1982, Seiten 749-55,
- CHEMICAL ABSTRACTS, vol. 92, no. 3, 21.Januar 1980 Columbus, Ohio, US; abstract no. 22191q, S.M. MAKIN ET AL.: "Chemistry of unsaturated ethers. XLVII. Synthesis and study of p-methoxyphenylpolyene aldehydes, their acetals and alkoxyacetals" Seite 656; XP002036895 & ZH. ORG. KHIM., Bd. 15, Nr. 9, 1979, Seiten 1852-6,
- ARCHIV DER PHARMAZIE, Bd. 316, Nr. 6, 1983, WEINHEIM, DE, Seiten 574-6, XP002036892 B. UNTERHALT ET AL.: "Notiz zur Darstellung substituierter Zimtaldehyde"
- JOURNAL OF ORGANIC CHEMISTRY, Bd. 53, Nr. 13, 24.Juni 1988, WASHINGTON, US, Seiten 2920-5, XP002036893 U. VON DER BRÜGGEN ET AL.: "Relative reactivities of acetals and orthoesters in Lewis acid catalyzed reactions with vinyl ethers. A systematic investigation of the synthetic potential of acetals and orthoesters in the electrophilic alkoxyalkylations of enol ethers"

## Beschreibung

Die vorliegende Erfindung betrifft Cinnamylidencampherderivate der Formel (1), 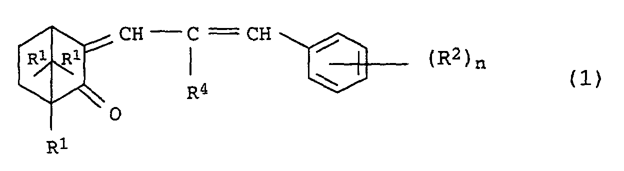 sowie dessen Verwendung in kosmetischen und pharmazeutischen Formulierungen.

Lichtschutzfilter auf der Basis von Campherderivaten sind bekannt. In DE 23 36 219 sind sulfonierte Benzyliden-und Cinnamylidencampherderivate beschrieben, die am Phenylring in der 4-Position unsubstituiert oder durch Methyl, Methoxy oder Chlor substituiert sind.

In DE 34 45 712 werden eine Reihe von ungesättigten Campherderivaten, bevorzugt Benzylidencampherderivate, beschrieben, die sich als Arzneimittel zur Behandlung von Hauterkrankungen und als Sonnenschutzmittel eignen.

In DE 44 26 216 werden Benzyliden-Norcampherderivate beschrieben, die als Sonnenfilter und zur Vorbeugung von Entzündungen und Hauterkrankungen einsetzbar sind.

In DE 44 24 489 wird ein Verfahren zur Herstellung von substituierten 4-Methylidenzimtsäurederivaten beschrieben.

Die Anforderungen, die an ein Lichtschutzmittel, das als UV-A-Filter eingesetzt werden soll, gestellt werden, sind zahlreich (Suncreens, ed. N.J. Lowe, N.A. Shaath, Marcel Dekker Inc., New York 1990, S. 230 - 231). Die wichtigsten sind:
1) Es hat sein Absorptionsmaximum im Bereich der UV-A-Region von 320 bis 360 nm;
2) es hat eine hohe spezifische Absorption in diesem Bereich;
3) es ist farblos, d. h. die Absorption oberhalb 400 nm sollte verschwindend gering sein, um eine Einfärbung des Hautschutzpräparates oder der Kleidung nach Anwendung auszuschließen;
4) es ist photo- und thermostabil;
5) es ist hautverträglich und führt nicht zu reizenden oder toxischen Wirkungen gegenüber der Haut;
6) es hat eine gute Haftung auf der Haut;
7) es ist mit kosmetischen Substanzen verträglich und in kosmetischen Lösungsmitteln und Zubereitungen gut löslich;
8) es ist isomerenrein

Die bekannten Cinnamylidencampherderivate sind Breitband-UV-Filter, die keine ausreichende Schutzwirkung im UV-A -Bereich entfalten. Ferner ist ihre Löslichkeit speziell in der Ölphase für einige Anwendungen nicht befriedigend.

Es bestand die Aufgabe, eine als UV-A Filter geeignete Verbindung auf bereitzustellen, die vor allem eine gute Photostabilität, gute Löslichkeit in der Ölphase und ein ausgeprägtes Absorptionsmaximum im UV-A Bereich aufweist.

Es wurde gefunden, daß Cinnamylidencampherderivate der Formel (1) 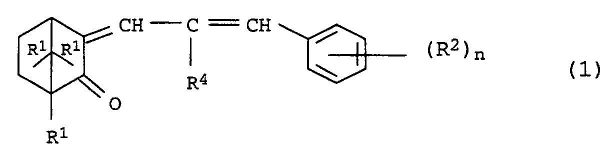 wobei das Diensystem in der Z;Z; Z,E; E,Z oder E,E Konfiguration vorliegt, und
R¹ = H, CH₃,
R² = C₄-C₆-Alkyl, n-Butoxy, tert.-Butoxy, NR³H, NR³₂,
R³ = C₁-C₄-Alkyl,
R⁴ = H, C₁-C₆-Alkyl,
n = 1, 2
bedeuten, mit der Maßgabe, daß wenn R² = n-Butoxy oder tert.-Butoxy, dann R¹ = CH₃, R⁴ = H, n = 1 bedeuten und R² in der 4- position steht.

die bestehende Aufgabe in hervorragender Weise lösen.

Die Herstellung von (1) erfolgt, indem Campher (2a), der racemisch oder in optisch aktiver Form, d. h. als definiertes Enantiomer oder als beliebige Mischung der Enantiomere vorliegen kann, oder Norcampher (2b), in Gegenwart einer Base gegebenenfalls unter Zusatz von einem oder mehreren Lösungsmitteln mit Zimtaldehyd (3), wie er nach EP 392 579 oder DE 3831713 zugänglich ist bei Temperaturen zwischen 0 °C und +200 °C umgesetzt wird. Der Zimtaldehyd (3) kann als Z- oder E-Isomeres der Doppelbindung bzw. als beliebige Mischung der geometrischen Isomeren eingesetzt werden.

Der Zimtaldehyd (3) kann auch durch eine Enoletherinsertion in Benzaldehydacetale hergestellt werden. Dazu wird das Benzaldehydacetal (4) mit einem Enolether (5) unter Lewis-Säure-Katalyse zu (6) umgesetzt, aus dem durch Hydrolyse der Zimtaldehyd (3) erhalten wird.

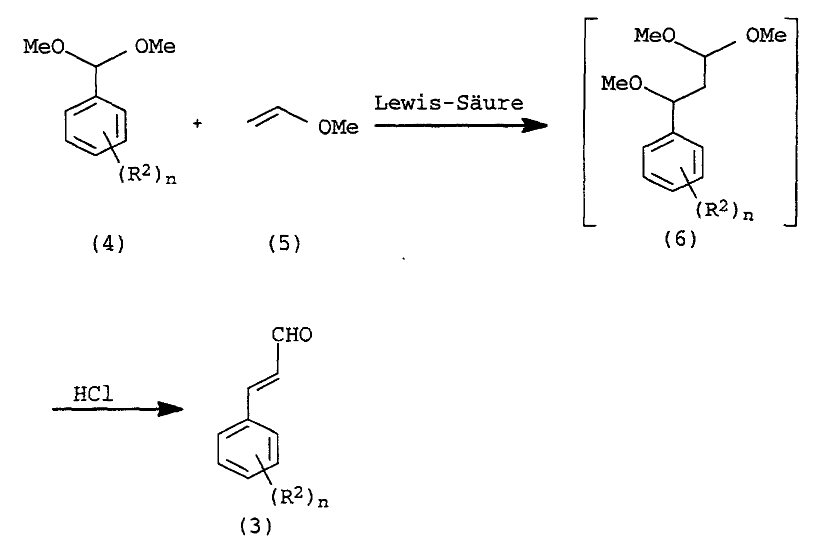

Für diese Reaktion setzt man Benzaldehydacetal (4) mit dem Enolether (5) in äquimolaren Mengen um. Als Katalysator wird bevorzugt das Bortrifluoridetherat in einer Menge von 0,001 - 0,01 Mol pro Mol Acetal (4) eingesetzt. Die Reaktion wird zwischen 0 und 60°C, bevorzugt zwischen 20 und 40°C durchgeführt.

Das erhaltene Insertionsprodukt (6) wird durch Hydrolyse, bevorzugt mit wäßriger Salzsäure, in den Zimtaldehyd (3) überführt.

Die erfindungsgemäße Verbindung (1) kann prinzipiell in ihren verschiedenen geometrischen Isomeren, d. h. mit einem Z,Z; Z,E; E,Z oder E,E-konfiguierten Diensystem, vorliegen. Besonders bevorzugt als kosmetisches Lichtschutzmittel ist das all-E-Isomere.

Die Umsetzung von (2) und (3) zu I ist in der Literatur unter dem Sammelbegriff "Aldolkondensation" bekannt (Organic Reactions 1968, Vol 16) und kann in an sich bekannter Weise durchgeführt werden.

Zur Herstellung der erfindungsgemäßen Verbindungen werden (2) und (3) 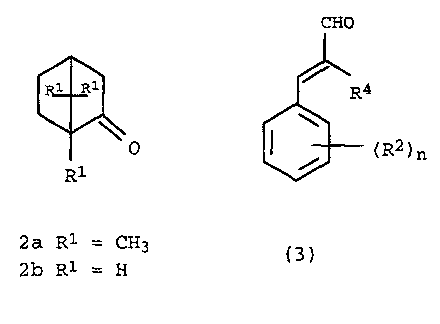 in Molverhältnissen von 0,1 : 1 bis 1 : 10 umgesetzt. Die Molverhältnisse können in weiten Grenzen variiert werden: Dabei ist ein Überschuß von (3) gegenüber (2) von Vorteil, da der Zimtaldehyd eher in Nebenreaktionen, z. B. wegen seiner Oxidationsempfindlichkeit, verbraucht wird. Besonders vorteilhaft ist die Verwendung eines 1,01-1,2fachen molaren Überschusses von (3) gegenüber (2), aber auch die Verwendung eines wesentlich höheren Überschusses ist nicht von Nachteil.

Die Reihenfolge, in der (2) und (3) zur Reaktion gebracht werden, ist beliebig. Die Base kann vorgelegt oder auch zudosiert werden. Die Reaktion ist auch als Eintopfreaktion durchführbar. Ebenso kann (2) zusammen mit Base vorgelegt und (3) zudosiert werden und umgekehrt. Auch ist es denkbar, die Base zusammen mit (3) vorzulegen und (2) zuzudosieren. Bevorzugt wird jedoch (2) zusammen mit der Base vorgelegt und (3) zudosiert.

Die zu verwendende Basenmenge kann im weiten Bereich relativ zu (2) variiert werden. Die molaren Verhältnisse Base zu (2) können 0,1 : 1 bis 1 : 20 betragen. Bevorzugt wird jedoch ein molarer Überschuß an Base verwendet. Besonders bevorzugt ist eine molare Menge an Base von 110 Mol-% gegenüber (2).

Als Base können beliebige organische oder anorganische Basen Verwendung finden, wie sie üblicherweise in Aldolreaktionen eingesetzt werden. Die Basen können im Reaktionsgemisch homogenisiert sein oder auch heterogen vorliegen. Es können auch Mischungen von zwei oder mehreren Basen benutzt werden. Als anorganische Basen können Alkyl- oder Erdalkalihydroxide, z. B. Natriumhydroxid, Kaliumhydroxid, Calciumhydroxid, Alkali- oder Erdalkalicarbonate, wie z. B. Natriumcarbonat, Kaliumcarbonat, Calciumcarbonat oder Alkali- oder Erdalkalihydride, wie z. B. Natriumhydrid oder Calciumhydrid, eingesetzt werden. Als organische Basen bieten sich Alkoxylate oder Carboxylate der Alkali- oder Erdalkalimetalle, wie z. B. Natriummethylat, Natriumethylat oder Natriumacetat, an. Aber auch Amine, wie z. B. Trimethylamin, Triethylamin, Triisopropylamin, Piperidin, Pyridin, DBU, Dabco oder basische Ionenaustauscher können Verwendung finden. Bevorzugt sind die Hydroxide der Alkalimetalle und Alkoxylate der Alkalimetalle. Besonders bevorzugt sind Kaliumhydroxid und Natriummethylat.

Die Reaktion wird im allgemeinen in aliphatischen Lösungsmitteln wie z. B. Toluol, Xylol, Hexan, Dibutylether oder Paraffingemischen mit C₁ bis C₂₀, durchgeführt. Die Reaktion läßt sich gleichfalls in protischen Lösungsmitteln, z. B. Ethanol, durchführen. Es ist aber auch möglich, die Reaktion in Substanz, d. h. ohne Lösungsmittel oberhalb der Schmelztemperatur des Gemisches durchzuführen. Bevorzugt wird in Toluol oder Paraffingemischen (C₅ bis C₁₅) gearbeitet. Es wird zur Erlangung einer guten Raumzeitausbeute in so wenig Lösungsmittel gearbeitet, daß bei Reaktionstemperatur eine rührbare Mischung entsteht. Eine größere Menge Lösungsmittel ist aber nicht von Nachteil.

Die Reaktion kann in einem weiten Temperaturintervall von 0 °C bis + 200 °C durchgeführt werden. Bevorzugt ist der Temperaturbereich von 20 °C bis 110 °C. Besonders bevorzugt ist der Temperaturbereich von 60 - 90 °C.

Die Reaktionszeit hängt unmittelbar von der Reaktionstemperatur ab. Im allgemeinen ist die Reaktion im besonders bevorzugten Temperaturbereich nach 1 bis 5 Stunden beendet. Bei niedrigeren Temperaturen oder geringen Stationärkonzentrationen kann sich die Reaktionszeit beträchtlich verlängern und bis zu 48 h in Anspruch nehmen.

Die Aufarbeitung erfolgt durch Hydrolyse mit Wasser, Eis oder Eiswasser in einem Temperaturbereich zwischen - 20 °C und 40 °C. Die bevorzugte Temperatur liegt zwischen - 5 °C und + 10 °C. Zur Neutralisation der überschüssigen Base kann jede beliebige Protonsäure, wie z. B. H₂SO₄, HCl, HCOOH, CH₃COOH, verwendet werden. Bevorzugt ist die Verwendung von verdünnter wäßriger Lösung mit einem Gehalt an Säure von 5 - 20 Gew.-%, beispielsweise 10 % Salzsäure.
Die Isolierung des Wertproduktes (1) erfolgt durch die üblichen Techniken, wie Sedimentieren, Filtrieren, Zentrifugieren, Phasentrennung und Extrahieren mit Lösungsmitteln, die zumindest in Gegenwart von Salzen mit Wasser nicht mischbar sind bzw. beschränkt wasserlöslich sind. Bevorzugt ist die Isolierung durch Phasentrennung nach der hydrolytischen Aufarbeitung. Dabei kann es vorteilhaft sein, dem Gemisch weiteres Lösungsmittel, das bei der Reaktion Verwendung gefunden hat, wie z. B. Toluol, zuzusehen, um die Phasentrennung zu verbessern oder ein Ausfallen des Produktes aus der organischen Phase zu verhindern. Als derartige Lösungsverbesserer kann auch jedes andere beliebige organische Lösungsmittel eingesetzt werden, in den das Wertprodukt eine ausreichende Löslichkeit besitzt und das mit Wasser nicht mischbar, bzw. nur beschränkt wasserlöslich ist.

Die Reinigung des Wertproduktes (1) kann durch Umkristallisation aus organischen Lösungsmitteln und deren Gemischen und/oder Wasser erfolgen. Bevorzugt sind Mischungen, die Alkohole enthalten.

Die Reinigung kann auch durch Ionenschmelzen und chromatographische Methoden aber auch durch Destillation erfolgen.

Die erfindungsgemäßen Verbindungen (1) können am Phenylring ein oder zwei Substituenten R² tragen, die im Falle von zwei Substituenten gleich oder verschieden sein können. Die Substitution erfolgt bevorzugt in der 2-, 4- oder 2- und 4-Position. Bevorzugte Verbindungen (1) sind solche, die durch R² einfach in der 4-Position substituiert sind.

Die erfindungsgemäßen Verbindungen (1) können aus einem Campherteil (R¹ = CH₃) oder einem Norcampherteil (R¹ = H) bestehen.

### Die Reste R² besitzen die folgende Bedeutung:

C₄-C₆-Alkyl, bevorzugt C₄-Alkyl wie n-Butyl, iso-Butyl, tert.-Butyl; n-Butoxy, tert.-Butoxy, primäres oder sekundäres Alkylamin NR³H bzw. NR³₂, wobei R³ für C₁-C₄-Alkyl steht; und n = 1 oder 2, mit der Maßgabe, daß wenn R² = n-Butoxy- oder tert.-Butoxy, dann R¹ = CH₃, R⁴ = H, n = 1 bedeuten und R² in der 4-Position steht.

Die Reste R⁴ bedeuten H, C₁-C₆-Alkyl, bevorzugt Methyl, Ethyl, n-Propyl, iso-Propyl, n-Butyl, iso-Butyl, tert.-Butyl, besonders bevorzugt H, Methyl und Ethyl.

Die erfindungsgemäßen Verbindungen (1) sind als Lichtschutzmittel, vor allem als UV-A Filter, für kosmetische und pharmazeutische Anwendungen besonders geeignet. Die UV-Filterwirkung kann aber auch zur Stabilisierung von Kunststoffen, Farbstofformulierungen oder Lacken ausgenutzt werden.

Kosmetische Präparate oder Zubereitungen enthalten die Verbindungen (1) im allgemeinen zu 0,1 bis 15 Gew.-%, vorzugsweise zu 5-10 Gew.%, bezogen auf die Formulierung, neben den in der Kosmetik üblichen Trägerstoffen oder Verdünnungsmitteln und gegebenenfalls üblichen kosmetischen Hilfsstoffen.

Von der Art des Trägers , Hilfsstoffes oder Verdünnungsmittel hängt es ab, ob das fertige lichtschutzmittelhaltige Präparat eine Lösung, ein Öl, eine Creme, eine Salbe, eine Lotion. ein Gel oder ein Pulver ist. Derartige Zubereitungen können beispielsweise der Zeitschrift "Seifen, Öle, Fette, Wachse", 1955, Seite 147 entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms.

Grundlage für Sonnenschutzöle sind beispielsweise pflanzliche Öle, wie Erdnußöl, Olivenöl, Sesamöl, Baumwollsamenöl, Kokosöl, Traubenkernöl, Ricinusöl oder Mineralöle, wie Vaselineöl, oder insbesondere flüssiges Paraffin, synthetische Fettsäureester und Glyceride. Grundlage für Salben sind beispielsweise Vaseline, Lanolin Eucerin oder Polyethylenglykole.

Grundlagen für Cremes sind beispielsweise fettreiche Cremes, Glycerin-, Polysaccharid-, Tylosecreme, für Cremes auf Basis von Fetten und Wachsen Cetylalkohol, Lanolincreme, Kakaobutter, Bienenwachs, Stearinsäure, Stearylalkohol, Glycerinmonostearat, native oder mineralische Öle und Fette.

Grundlagen für Emulsionen sind beispielsweise Mischungen aus Stearylglykol, einem pflanzlichen und/oder Mineralöl, wie Mandelöl, Paraffinöl und Vaseline, und Wasser oder Mischungen aus Ethylalkohol, Wasser, Lanolin und Tragant, oder Mischungen aus Ethylalkohol, Stearin, Wasser, Tragant und Glycerin oder Mischungen aus Stearinsäure, Paraffinöl, Propyl- oder Isopropylalkohol und Wasser.

Die erfindungsgemäßen Verbindungen können als einzige UV-Absorber in den entsprechenden Zubereitungen eingesetzt werden; man kann sie jedoch auch in Kombination mit anderen UV-Absorbern- insbesondere UV-B-Absorbern einsetzen.

Beispiele für solche Verbindungen sind p-Aminobenzoesäureethylester (25 Mol) ethoxyliert, p-Methoxyzimtsäure-2-ethylhexylester, 2-Ethylhexyl-2-cyano-3,3-diphenylacrylat, 2-Phenylbenzimidazolsulfonsäure und Salze, 2-Hydroxy-4-methoxybenzophenon, 2-Hydroxy-4-methoxybenzophenon-5-sulfonsäure, 3-(4'-Methylbenzyliden)-d,l-campher, 2,4,6-Trianilino-(p-carbo-2'-ethylhexyl-1'-osy)-1,3,5-triazin.

### Beispiel 1

### Herstellung von 4 tert. Butylcinnamylidencampher (1)

In einem 500 ml Vierhalskolben mit Thermometer, Rückflußkühler und Rührer wurden unter Stickstoff 31,7 g (0,2 mol) D,L-Campher (96 %) in 40 g Toluol gelöst. Nach der Zugabe von 12,1 g (0,22 mol) Natriummethylat und Erwärmen auf 70 °C entstand eine gelbe Suspension. Es wurde 0,5 h bei 70 °C nachgerührt.

Daraufhin wurden innerhalb von 3,25 h 42,2 g (0,21 mol) 4-tert.-Butylzimtaldehyd in 120 g Toluol gelöst zugetropft. Die Farbe der Suspension verdunkelte sich zu einer orangen Tönung. Es wurde 0,5 h bei Temperatur nachgerührt.

Zur Aufarbeitung wurde der Ansatz mit 200 g destilliertem Wasser versetzt, aufgerührt und eine Phasentrennung durchgeführt. Die organische Oberphase wurde erneut mit 100 g Wasser versetzt und bei 50 °C mit 3 % wäßriger Schwefelsäure ein pH-Wert von 5 eingestellt. Es wurden erneut die Phasen getrennt, um daraufhin das Lösungsmittel in Vakuum abzudestillieren.

Der Destillationsrückstand wurde aus Methanol umkristallisiert. Die gelblichen Kristalle wurden mit 130 ml Methanol/Wasser 1 : 1 gewaschen und bei 50 °C getrocknet.

Man erhielt 22,3 g analysenreine Kristalle.
Schmelzpunkt: 98 °C
UV-Spektrum (Methanol): λₘₐₓ₁: 338,1 nm

| | |
|---|---|
| Elementaranalyse: | C 85,8 % (Theorie 85,7 %) |
| | H 9,0 % (Theorie 9,4 %) |
| | O 5,0 % (Theorie 5,0 %) |

| | |
|---|---|
| 1H-NMR (CDCl₃): | 7,4 ppm (Quartett, 4 H) |
| | 6,95 ppm (Multiplett, 3 H) |
| | 2,95 ppm (Dublett, 1 H) |
| | 1,40-2,10 ppm (Multiplett, 4 H) |
| | 1,3 ppm (Singlett, 9 H) |
| | 1,00 ppm (zwei Singletts, 6 H) |
| | 0,85 ppm (Singlett,3 H) |

### Beispiel 2

### Herstellung von 4-n-Butoxycinnamylidencampher

In einem 500 ml Vierhalskolben mit Thermometer, Rückflußkühler und Rührer wurden unter Stickstoff 31,7 g (0,2 mol) D,L-Campher (96 %) in 30 g Toluol gelöst. Nach Zugabe von 12,1 g (0,22 mol) Natriummethylat und Erwärmen auf 70 °C entstand eine gelbe Suspension. Es wurde 0,5 h bei 70 °C nachgerührt.

Daraufhin wurden innerhalb von 3 h 44,5 g (0,21 mol) 4-n-Butoxyzimtaldehyd gelöst in 40 g Toluol zugetropft und 0,5 h bei 70°C nachgerührt

Zur Aufarbeitung wurde der Ansatz mit 200 g destilliertem Wasser versetzt, aufgerührt und eine Phasentrennung durchgeführt. Die organische Oberphase wurde erneut mit 100 g Wasser versetzt und bei 50 °C mit 3 % wäßriger Schwefelsäure auf pH 3 eingestellt. Es wurden erneut die Phasen getrennt und daraufhin das Lösungsmittel bei 110°C und 1 mbar abdestilliert.

Der rote Rückstand wurde aus 140 Ethanol umkristallisiert. die Kristalle mit kaltem Ethanol gewaschen und bei 50 °C getrocknet.

Man erhielt 35 g analysenreine Kristalle.
Schmelzpunkt: 108-109 °C
UV-Spektrum: λₘₐₓ₁: 353 nm

| | |
|---|---|
| Elementaranalyse: | C 81,5 % (Theorie 81,61 %) |
| | H 8,9 % (Theorie 8,93 %) |
| | O 9,3 % (Theorie 9,45 %) |

### Beispiel 3

Analog zu Beispiel 1 und 2 wurden die in Tab. 1 aufgeführten Verbindungen 1.1-1.15 hergestellt

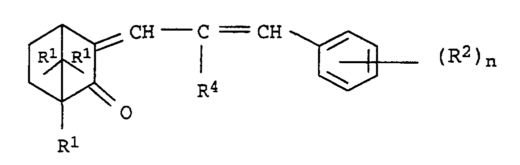

**Tabelle 1**

| Verbind. | R¹ | R² | R⁴ |
|---|---|---|---|
| 1.1 * | CH₃ | 2-OCH₃ | H |
| 1.2 * | CH₃ | 2-CH₃ | H |
| 1.3 | CH₃ | 4-N(CH₃)₂ | H |
| 1.4 | CH₃ | 4-tert.-Butoxy | H |
| 1.5 * | CH₃ | 4-OCH₃ | H |
| 1.6 * | CH₃ | 4-CH₃ | H |
| 1.7 | CH₃ | 4-n-Butyl | H |
| 1.8 * | CH₃ | 4-i-Propyl | H |
| 1.9 | H | 4-tert.-Butyl | H |
| 1.10 * | CH₃ | 4-OCH₃ | CH₃ |
| 1.11 * | CH₃ | 4-CH₃ | CH₃ |
| 1.12 | CH₃ | 4-tert.-Butyl | CH₃ |
| 1.13 * | CH₃ | 4-CH₃ | CH₂-CH₃ |
| 1.14 * | CH₃ | 4-OCH₃ | CH₂-CH₃ |
| 1.15 | CH₃ | 4-tert.-Butyl | CH₂-CH₃ |

| | | | |
|---|---|---|---|
| * nicht erfindungsgemäß | | | |

## Patentansprüche

1. Cinnamylidencampherderivate der Formel (1) 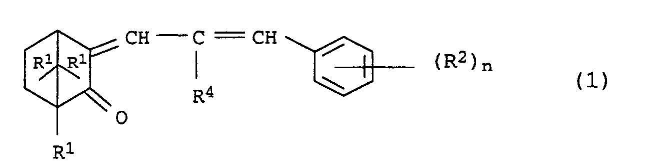 wobei das Diensystem in der Z,Z; Z,E; E,Z oder E,E Konfiguration vorliegt und
R¹ = H, CH₃,
R² = C₄-C₆-Alkyl, n-Butoxy, tert.-Butoxy, NR³H, NR³₂,
R³ = C₁-C₄-Alkyl,
R⁴ = H, C₁-C₆-Alkyl,
n = 1, 2
bedeuten, mit der Maßgabe, daß wenn R² = n-Butoxy oder tert.-Butoxy, dann R¹ = CH₃, R⁴ = H, n = 1 bedeuten und R² in der 4-Position steht.

2. Verbindung nach Anspruch 1, **dadurch gekennzeichnet, daß** das Diensystem in der E,E Konfiguration vorliegt.

3. Verbindung nach Anspruch 1, **dadurch gekennzeichnet, daß** R¹ = CH₃, R² = tert. Butyl, n = 1 bedeuten und R² in der 4-Position steht.

4. Verwendung einer Verbindung nach Anspruch 1 - 3 als Lichtschutzmittel.

5. Verwendung nach Anspruch 4 in kosmetischen oder pharmazeutischen Formulierungen.

## Claims

1. A cinnamylidenecamphor derivative of the formula (1) 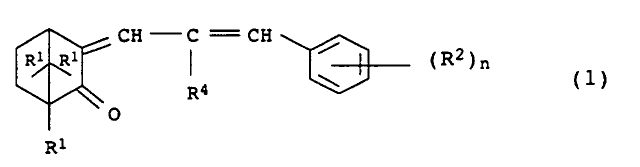 where the diene system is in the Z,Z, Z,E, E,Z or E,E configuration, and
R¹ is H, CH₃,
R² is C₄-C₆-alkyl, n-butoxy, tert-butoxy, NR³H, NR³₂,
R³ is C₁-C₄-alkyl,
R⁴ is H, C₁-C₆-alkyl,
n is 1, 2,
with the proviso that if R² is n-butoxy or tert-butoxy, then R¹ is CH₃, R⁴ is H, n is 1 and R² is in position 4.

2. A compound as claimed in claim 1, wherein the diene system is in the E,E configuration.

3. A compound as claimed in claim 1, wherein R¹ is CH₃, R² is tert-butyl, n is 1 and R² is in position 4.

4. The use of a compound as claimed in claim 1-3 as sunscreen agent.

5. The use as claimed in claim 4 in cosmetic or pharmaceutical formulations.

## Revendications

1. Dérivés du cinnamylidènecamphre de formule 1 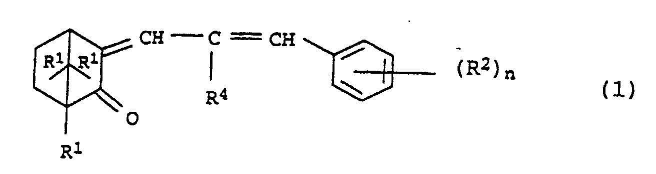 dans laquelle le système diénique est en configuration Z,Z ; Z,E ; E,Z ou E,E et
R¹ = H, CH₃,
R² = alkyle en C4-C6, n-butoxy, tert-butoxy, NR³H, NR³₂,
R³ = alkyle en C1-C4,
R⁴ = H, alkyle en C1-C6,
n = 1, 2
sous réserve que lorsque R² représente un groupe n-butoxy ou tert-butoxy, R¹ représente CH₃, R⁴ représente H, n est égal à 1 et R² est en position 4.

2. Composé selon la revendication 1, **caractérisé par le fait que** le système diénique est en configuration E,E.

3. Composé selon la revendication 1, **caractérisé par le fait que** R¹ = CH₃, R² = tert-butyle, n = 1 et R² est en position 4.

4. Utilisation d'un composé selon les revendications 1 à 3 en tant qu'agent de protection contre la lumière.

5. Utilisation selon la revendication 4 dans des compositions cosmétiques ou pharmaceutiques.
